Europäisches Patentamt

European Patent Office

Office européen des brevets

(11) Publication number : **0 529 893 A1**

# EUROPEAN PATENT APPLICATION

(21) Application number : **92307460.3**

(51) Int. Cl.⁵ : **A23L 1/09, C08B 30/20**

(22) Date of filing : **14.08.92**

(30) Priority : **16.08.91 US 746381**
**26.11.91 US 798291**

(43) Date of publication of application :
**03.03.93 Bulletin 93/09**

(84) Designated Contracting States :
**AT BE CH DE DK ES FR GB GR IE IT LI LU MC NL PT SE**

(71) Applicant : **A.E. STALEY MANUFACTURING COMPANY**
**2200 E. Eldorado Street**
**Decatur Illinois 62525 (US)**

(72) Inventor : **Harris, Donald W.**
**3333 Lost Bridge Road**
**Decatur, Illinois (US)**
Inventor : **Little, Jeanette A.**
**1421 Riverview Street**
**Decatur, Illinois (US)**

(74) Representative : **Cockbain, Julian Roderick Michaelson**
**Frank B. Dehn & Co. European Patent Attorneys Imperial House 15-19, Kingsway London WC2B 6UZ (GB)**

(54) Debranched amylopectin-starch as fat replacer.

(57) A method of preparing reduced fat foods is provided which employs a debranched amylopectin starch. A debranched amylopectin starch is dissolved and then allowed to form an aqueous dispersion of particles that is useful in replacing fat in a variety of food formulations. The debranched amylopectin starch can be derived from a starch which contains amylopectin, e.g. common corn starch and waxy maize starch, by gelatinizing the starch followed by treatment with a debranching enzyme, e.g. isoamylase or pullulanase and precipitation of the debranched starch.

EP 0 529 893 A1

This invention relates to food formulations in which at least a portion of the fat and/or oil is replaced by a carbohydrate.

U.S. Patent No. 4,510,166 (Lenchin et al.) discloses converted starches having a DE less than 5 and certain paste and gel characteristics which are used as a fat and/or oil replacement in various foods, including ice cream and mayonnaise. The converted starches are described as dextrins, acid-converted starches (fluidity starches), enzyme-converted starches and oxidized starches. It is also disclosed that if the converted starches are not rendered cold-water soluble by the conversion, they are pregelatinized prior to use or cooked during use.

A product bulletin entitled "Paselli SA2; The Natural Alternative to Fats and Oils" (AVEBE b.a., Foxhol, Holland, Ref. No. 05.12.31.167 EF) discloses the use of a low-DE-hydrolysate (DE less than 3) made from potato starch as a replacement for fifty percent of the fat with an amount of the low-DE-potato starch hydrolysate plus water (starch hydrolysate at 28% dry solids) equal to the amount of fat replaced.

U.S. Patent Nos. 3,962,465 (Richter et al.) and 3,986,890 (Richter et al.) disclose the use of thermoreversible gels of a starch hydrolysate (formed by enzymatic hydrolysis) as a substitute for fat in a variety of foods, including cake creams and fillings, mayonnaise and remoulades, cream cheeses and other cheese preparations, bread spreads, pastes, meat and sausage products, and whipped cream.

U.S. Patent No. 4,971,723 (Chiu) discloses partially debranched starch prepared by enzymatic hydrolysis of the $\alpha$-1,6-D-glucosidic bonds of the starch, comprising amylopectin, partially debranched amylopectin and up to 80% by weight, short chain amylose and that the partially debranched starch is useful in a variety of ways depending upon the degree of debranching. It is disclosed that a waxy maize starch (or other waxy starch) can be partially debranched (i.e. to 25% to 70% short chain amylose) to yield sufficient short chain amylose to form a thermally reversible gel in an aqueous starch suspension. It is further disclosed that the same degree of debranching of waxy starches is preferred for lending a fat-like, lubricating texture to an aqueous starch dispersion.

In one aspect, this invention relates to a food formulation having a reduced level of fat and/or oil comprising a mixture of a foodstuff and a particle gel comprising a minor amount of debranched amylopectin starch particles dispersed within a major amount of an aqueous liquid as a replacement for at least a substantial portion of the fat and/or oil of said food formulation.

In another aspect, this invention relates to a method of formulating a food containing a fat and/or oil ingredient comprising replacing at least a substantial portion of said fat and/or oil ingredient with a particle gel comprising a minor amount of debranched amylopectin starch particles dispersed within a major amount of an aqueous liquid.

By "debranched amylopectin starch particles" is meant a starch material comprised of amylopectin which has been subjected to enzymatic debranching followed by dissolution in an aqueous liquid and then particle formation therein to form a particle gel. The debranching and dissolution will be sufficient to produce particles which will form an aqueous dispersion having the characteristics of a particle gel.

In another aspect this invention relates to a method of making a composition of matter useful in replacing fat and/or oil in a food formulation comprising dissolving a minor amount of a debranched amylopectin starch in a major amount of an aqueous liquid and then forming particles of said debranched amylopectin starch dispersed in said aqueous liquid, said dissolving and forming being effective to form a particle gel of said composition.

In another aspect, this invention relates to an aqueous dispersion useful as a replacement for fats and/or oils comprising a particle gel comprised of a major amount by weight of water and a minor amount by weight of debranched amylopectin starch particles dispersed therein.

The terms "foodstuff" and "food", as used herein, are intended to broadly cover nutritional and/or functional materials that are ingested by humans in the course of consuming edible fare. The term "fats and/or oils" is intended to broadly cover edible lipids in general, specifically the fatty acid triglycerides commonly found in foods. The terms thus include solid fats, plastic shortenings, fluid oils (and fully or partially hydrogenated oils), and the like. Common fatty acid triglycerides include cottonseed oil, soybean oil, corn oil, peanut oil, canola oil, sesame oil, palm oil, palm kernel oil, menhaden oil, whale oil, lard, and tallow. The technology of fats and/or oils is described generally by T. H. Applewhite, "Fats and Fatty Oils", Encyclopedia of Chemical Technology, Vol. 9, pp. 795-831 (Kirk-Othmer, eds., John Wiley & Sons, Inc., New York, New York, 3d ed., 1980), the disclosure of which is incorporated by reference.

The use of the terms "major" and "minor" in context together in this specification is meant to imply that the major component is present in a greater amount by weight than the minor component, and no more nor less should be inferred therefrom unless expressly noted otherwise in context.

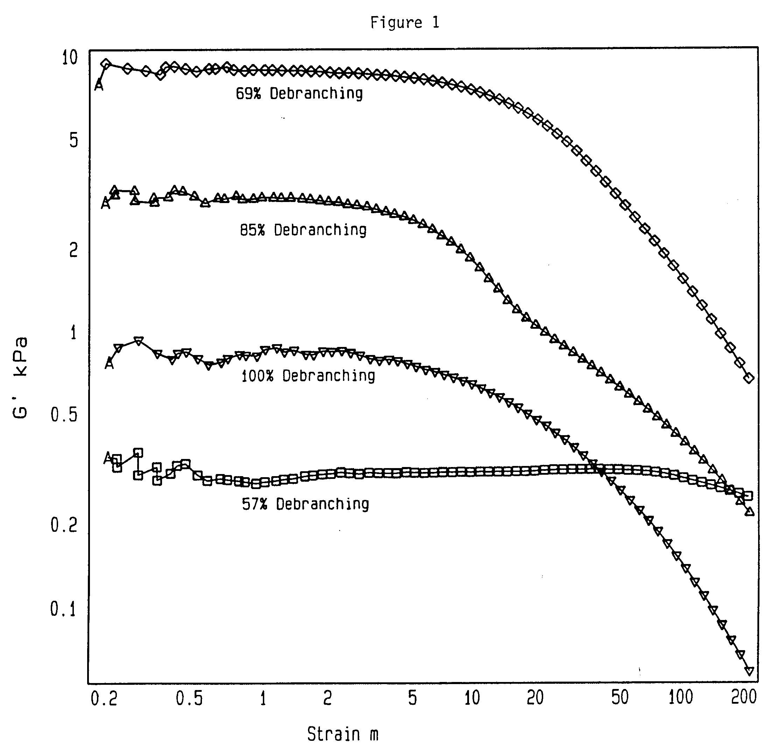

Figure 1 shows the dynamic elastic modulus (G′) in kilo pascals as a function of strain in millistrain for debranched amylopectin starch precipitates at 15% precipitate solids having different degrees of debranching.

The debranched amylopectin starch particles are made by the sequential steps of debranching, dissolution, and particle formation of a starch material containing amylopectin. Starch is generally comprised of a highly-branched glucan having $\alpha$-1,4 and $\alpha$-1,6 linkages, denominated amylopectin, and a substantially linear glucan, having almost exclusively $\alpha$-1,4 linkages, denominated amylose. Methods of determining the amounts of each are referenced in R.L. Whistler et al., Starch: Chemistry and Technology, pp. 25-35 (Academic Press, Inc., New York, New York, 1984), the disclosure of which is incorporated by reference. Starches having a major proportion (i.e. at least 50% by weight) of amylopectin are preferred and examples of these include the common non-mutant starches of, cereals, tubers and legumes, e.g. corn, wheat, rice, potato and tapioca, pea and mutant varieties comprised of a major proportion of amylopectin, e.g. waxy maize. Common starches derived from corn (Zea mays) such as corn starch and waxy maize starch, each of which are examples of starches containing less than 40% amylose, are useful herein. However, starches containing a major amount of amylose (e.g. 50% to 75% by weight) are also useful and may be preferred depending upon the precise properties desired in the final product. Examples of such starches from high amylose corn include HI-SET® C and HYLON™ (each about 55% amylose by weight) and HYLON™ VII (about 70% amylose by weight), all available from National Starch and Chemical Corporation, Bridgewater, New Jersey.

In certain embodiments, the starch consists essentially of amylopectin. In such embodiments, the starch employed is from a mutant variety of native starch which consists essentially of amylopectin or is amylopectin derived from a native starch variety containing both amylose and amylopectin. Methods for the fractionation of amylose and amylopectin from native starch are disclosed in, for example, U.S. Patent No. 3,067,067 (Etheridge).

If the starch chosen as a starting material is not in pre-gelatinized or instant form, the starch must be gelatinized or pasted prior to debranching. The gelatinization or pasting process disrupts, at least in substantial part, the associative bonding of the starch molecules in the starch granule. This permits the enzyme access to the molecule to more easily and uniformly debranch the amylopectin molecules. This disruption is accomplished by heating a slurry of the starch to a sufficient temperature for a sufficient length of time depending upon the inherent resistance of the particular starch to gelatinization and the amount of moisture present in the slurry. The slurry will typically be comprised of a major amount of water (i.e. at least 50% by weight) and a minor amount of the starch starting material (i.e. less than about 50% by weight). Preferably, the starch slurry will contain at least about 5% starch, typically between about 10% to about 25% starch. The pH of the slurry will generally be substantially neutral, i.e. from about 3.5 to about 9 and more preferably from about 6 to 8, to minimize hydrolysis of the starch molecules. The time, temperature, slurry solids and pH should be optimized to gelatinize the starch, yet minimize hydrolysis of the starch.

The appropriate temperature, pressure and period of treatment needed to provide a starch paste is preferably obtained by processing aqueous starch slurries in equipment commonly known in the art as steam injection heaters or jet cookers. In such equipment, superatmospheric steam is injected and mixed with a water slurry of starch in a throat section of a jet. Upon contact with the injected steam, the starch granules are uniformly and thermally treated under turbulent conditions whereupon the starch granules are gelatinized and solubilized. Examples of steam injection heaters wherein the temperature, pressure and feed rate can be regulated to provide the desired starch pastes are disclosed in U.S. Patent Nos. 3,197,337; 3,219,483; and 3,133,836. More uniformly solubilized starch pastes are obtained by use of the steam injection heater in combination with a holding zone such as coiled tubing or a pressurized tank constructed to minimize liquid channeling. Other pasting equipment, e.g. heat exchangers, homogenizers, cookers, votators, sizeometer cookers, kettle cookers, etc., may be employed provided the pasting conditions can be adequately maintained.

The gelatinized starch is then treated with a debranching enzyme, i.e. an enzyme capable of hydrolyzing the 1,6-glucosidic bond of amylopectin without significant capability of hydrolyzing the 1,4-glucosidic bond. Enzymes from a variety of sources are capable of debranching amylopectin. U. S. Patent No. 3,370,840 (Sugimoto et al.) describes sources of debranching enzymes, the disclosure of which is incorporated herein by reference. Examples of useful enzymes include pullulanases derived from bacteria of the genus Aerobacter (e.g. E.C. 3.2.1.41 pullulan 6-glucanohydrolase) and isoamylases derived from bacteria of the genus Pseudomonas (e.g. E.C. 3.2.1.68 glycogen 6-glucanohydrolase). Particularly useful enzymes include thermostable enzymes, e.g. thermostable pullulanases as disclosed in PCT Publ. No. WO 92/02614, published February 20, 1992, the disclosure of which is incorporated by reference, and which are obtained from members of the genus Pyrococcus. The debranching enzyme may be in solution during debranching or it may be immobilized on a solid support.

The debranching enzyme preparation should be as specific as possible for the hydrolysis of the 1,6-glucosidic bond of amylopectin and amylose. Thus, the enzyme preparation, if it contains a mixture of enzymes, is preferably essentially free of enzymes capable of hydrolyzing $\alpha$-1,4-glucosidic bonds. Minimizing hydrolysis of $\alpha$-1,4-glucosidic bonds will help to minimize the amounts of dextrose and soluble oligomers produced during debranching. Because these soluble saccharides are not believed to contribute to the functionality of the debranched material, minimizing their production will enhance the yield of functional material.

The debranching enzyme is allowed to act upon the solubilized starch containing amylopectin. The optimum concentration of enzyme and substrate in the debranching medium will, in general, depend upon the level of activity of the enzyme which, in turn, will vary depending upon the enzyme source, enzyme supplier and the concentration of the enzyme in commercial batches. When the isoamylase E.C. 3.2.1.68, derived from Pseudomonas amyloderamosa, available from Sigma Chemical Co., St. Louis, Missouri, is employed, typical conditions include the treatment of a starch solution at 5% to 30% by weight starch solids with about 50 units of enzyme per gram of starch for a period of about 48 hours to obtain substantially complete debranching.

The optimum pH and temperature of the debranching medium will also depend upon the choice of enzyme. The debranching medium may, in addition to the water used to solubilize the starch, contain buffers to ensure that the pH will be maintained at an optimum level throughout the debranching. Examples of useful buffers include acetates, citrates, and the salts of other weak acids. With the isoamylase described above, the pH is preferably maintained at about 4.0 to 5.0 and the temperature from about 40°C to about 50°C. With the thermostable pullulanase described above, the pH is preferably maintained between 5 and 7 and the optimum temperature should be between 85°C and 115°C.

The debranching is allowed to proceed until the desired degree of debranching has been obtained. The precise degree of debranching needed to obtain the desired particle gel of the debranched amylopectin starch may vary depending upon the source of the starch and the precise properties desired in the resulting gel. Preferably, the degree of debranching is sufficient to convert more than about 80% of the amylopectin in the starch to short chain amylose and, more preferably, at least about 90% of the amylopectin.

In preferred embodiments, essentially all (i.e. greater than 99% by weight) of the amylopectin is converted to short chain amylose. The amount of short chain amylose can be measured by gel permeation chromatography as set forth in U.S. Patent No. 4,971,723, wherein short chain amylose is calculated from the relative area of the peak obtained within the molecular weight range of 500 to 20,000. Thus, preferably less than 20% of the amylopectin that was originally present will be present as molecular species having a molecular weight in excess of 20,000 g/mol, and most preferably, essentially no amylopectin having a molecular weight in excess of 20,000 g/mol will remain. (It should be noted that if amylose is present, at least a portion thereof may be debranched to produce molecules above the 20,000 g/mol cut-off and molecules below the 20,000 g/mol cut-off. To measure how much of the material eluting between 500 g/mol and 20,000 g/mol is debranched amylopectin and how much is debranched amylose, it may be necessary to fractionate the starting starch into its amylose and amylopectin fractions and then debranch and elute each fraction separately.)

It has been found, as described more fully below, that when waxy maize starch was debranched, dissolved, and then allowed to form dispersed particles, a degree of debranching of only about 57% resulted in an aqueous dispersion that displayed the rheological characteristics of a polymer gel. However, when the debranching was allowed to proceed to 69%, 85%, or 100%, an aqueous dispersion of the starch particles displayed rheological properties more characteristic of a particle gel, with the 85% and 100% debranched samples exhibiting more particle gel character than the 69% debranched samples.

After the desired degree of debranching is obtained, debranching enzyme in solution is deactivated, e.g. by heating to denature the enzyme. If an immobilized enzyme is employed, the contact time of the solubilized starch is adjusted so that the starch is removed from the enzyme when the desired degree of debranching is obtained. The debranching medium may be concentrated by removal of water therefrom, e.g. by evaporation, to facilitate precipitation. The debranching medium may also be treated to remove impurities therefrom. Treatment with, for example, activated carbon will remove residual proteins and lipids that may contribute to off-flavors and/or colors.

The solution of debranched starch is then typically treated to isolate the debranched amylopectin starch therefrom. Generally, the solution will be cooled, e.g. to ambient temperature, to reduce the solubility of the debranched starch therein and thereby cause the debranched amylopectin starch to form a precipitate. The solution will typically be allowed to stand until substantial equilibrium is achieved between the supernatant and the precipitate. The precipitate can be isolated from the supernatant, e.g. by centrifugation, but isolation from the supernatant is not necessary to form a useful product.

The isolated debranched amylopectin starch precipitate is typically washed and then dried (e.g. to a low moisture content, typically 3-12%) after isolation to allow for handling and storage prior to further processing. Examples of drying techniques include spray drying, flash drying, tray drying, belt drying, and sonic drying. The dried precipitate may be hygroscopic. Thus, some rehydration during handling and storage may occur. Depending upon the precise composition of the precipitate and the conditions (including length of time) of storage, steps to maintain the moisture at a low content may be necessary (e.g. moisture barrier packaging and/or control of humidity in the storage environment). If the moisture content is allowed to rise too far (e.g. greater than about 20%, or possibly greater than 15%), bulk handling problems and/or microbiological stability problems might arise.

The debranched amylopectin starch precipitate may also be otherwise chemically modified. Examples of such chemical modification include the product of reaction with bleaching agents (e.g. hydrogen peroxide, peracetic acid, ammonium persulfate, chlorine (e.g. calcium and/or sodium hypochlorite or sodium chlorite), and permanganate (e.g. potassium permanganate)); esterifying agents (e.g. acetic anhydride, adipic anhydride, octenyl succinic anhydrides, succinic anhydride, vinyl acetate); including phosphorous compounds (e.g. monosodium orthophosphate, phosphorous oxychloride, sodium tripolyphosphate, and sodium trimetaphosphate); and/or etherifying agents (e.g. acrolein, epichlorohydrin, and/or propylene oxide), provided such chemical modification will not prevent dissolution of the debranched starch and formation of debranched starch particles. Such chemical modifications will typically be accomplished after the debranching step, but may be accomplished prior to the debranching or effected by using a modified starch as a starting material for the debranching step, provided such modification does not preclude debranching.

It is contemplated that commercial production and use may involve hydrolysis, and drying (e.g. spray drying) of the starch precipitate to produce an item of commerce. This item of commerce will then be purchased by a food processor for use as an ingredient. To incorporate the dried, debranched amylopectin starch precipitate into a food product, it will be necessary to dissolve the starch precipitate and cause it to reprecipitate to form a gel either in situ in a foodstuff or as a separate gel which can be dispersed into the foodstuff in which it will be employed.

The debranched amylopectin starch is used to form a particle gel by dissolution in an aqueous liquid followed by the formation of particles. Dissolution is generally accomplished by mixing the debranched amylopectin starch with a major amount by weight of an aqueous medium, preferably tap water, and holding the mixture at a temperature which will cause at least a major amount by weight of the starch to dissolve (i.e. at least 50% by weight). Although tap water is the preferred liquid medium for the dissolution of the debranched starch, other liquids are suitable provided sufficient water is present to hydrate the particles of starch which form and, thus, result in a dispersion having the characteristics of a particle gel. Sugar solutions, polyols, of which glycerol is an example, alcohols, particularly ethanol, isopropanol, and the like, are good examples of suitable liquids that can be in admixture with water in the liquid medium.

The amount of water will be sufficient to dissolve at least a major portion by weight of the debranched starch at an elevated temperature (e.g. 90°C). The ratio of water to debranched amylopectin starch solids will typically range from about 19:1 to about 1:1, more typically 9:1 to about 2:1. The dissolution of the debranched amylopectin starch in the liquid medium will be facilitated by simple mixing of the debranched amylopectin starch and liquid medium. Heat can be applied to the mixture and/or the liquid medium can be preheated, as desired, to cause the debranched starch to dissolve.

The debranched amylopectin starch is dissolved under conditions such that the molecular fragments of debranched starch will associate to form particles. These particles form a dispersed solid phase within the aqueous media. The formation of the particles can be considered precipitation of the debranched starch from solution, but the particles remain dispersed in the aqueous medium and do not settle out to an appreciable degree over any practical period. The dissolution and particle formation can be accomplished in a number of somewhat different ways. In certain embodiments, the debranched amylopectin starch is dissolved in the aqueous medium in which it will form a particle gel. The resulting solution is then cooled or allowed to cool, e.g. to ambient temperature, to allow the debranched starch to associate to form dispersed particles.

In other embodiments, the debranched amylopectin starch will be dissolved in an aqueous medium which is removed (e.g. evaporated by spray drying) before the debranched amylopectin starch can associate to form the dispersed particles. The resulting dried debranched amylopectin starch can then be stored as a stable dry powder prior to gel formation. Gel formation is then accomplished by dissolution of the dry powder in a liquid medium followed by association of the debranched starch to form dispersed particles.

The debranched amylopectin starch particles which make up the particle gel can be analyzed in a variety of ways. Rheological measurements can be made to determine the rheological characteristics of the resulting dispersion. Typically, the aqueous dispersion of debranched amylopectin starch particles will exhibit a transition in dynamic elastic modulus (G') versus shear strain at less than about 50 millistrain, and preferably less than about 10 millistrain, said transition being from a substantially constant G' versus shear strain to a decreasing G' versus shear strain. The transition indicates fracture of the particle network within the particle gel and is typically a sharp transition.

Analysis of the debranched amylopectin starch particles formed after dissolution shows that the starch has a measurable crystallinity. The crystalline regions of particles derived from fully debranched waxy maize starch (essentially no amylose component) exhibit a diffraction pattern characteristic of a starch material consisting essentially of A-type starch crystals. The crystalline regions of particles derived from substantially fully debranched common corn starch (about 28% amylose) exhibit a diffraction pattern characteristic of a starch material consisting essentially of B-type starch crystals.

Heating (e.g. to about 70°C) of the particles while in contact with the aqueous medium to dissolve at least a portion of the mass of the particles and then cooling of the suspension/solution can also be employed in forming the particle gel of this invention. This heating to an elevated temperature and then reformation of the particles tends to make the particles resistant to melting or dissolving when an aqueous dispersion of the particles is exposed to heat in processing, e.g. in a pasteurization step. In general, the higher the temperature to which the particles in the liquid medium are heated (and thus the greater the amount of precipitate that is redissolved), the higher the temperature at which the resulting aqueous dispersion of the particles will be stable. Repetition of the dissolving and reformation may improve the temperature stability of the resulting aqueous dispersion. Further, a slow rate of heating and/or cooling (e.g. from about 0.005°C/min. to about 0.5°C/min., for each) may be advantageous.

The use of the debranched amylopectin starch particles allows for the replacement of a substantial portion (e.g. from 10% to 100% by weight) of the fat and/or oil in a food formulation. The precise level of replacement that is possible without significantly decreasing the organoleptic quality of the food will generally vary with the type of food. For example, in a French-style salad dressing, it is generally possible to completely replace the oil component that is normally present. In other types of foods, e.g. frostings, icings, cream fillings, ice cream, margarine, etc., a major amount of the fat and/or oil (e.g. about 50% to about 80%) can be replaced with little effect on the organoleptic desirability of the food. Examples of typical foods in which fat and/or oil can be replaced include frostings (e.g. icings, glazes, etc.), creme fillings, frozen desserts (e.g. ice milk, sherbets, etc.), dressings (e.g. pourable or spoonable salad and/or sandwich dressings), meat products (e.g. sausages, processed meats, etc.), cheese products (e.g. cheese spreads, processed cheese foods), margarine, fruit butters, other imitation dairy products, puddings (e.g. mousse desserts), candy (e.g. chocolates, nougats, etc.), and sauces, toppings, syrups and so on.

Generally, it will be desirable to remove sufficient fat from a given food formulation to achieve a reduction in calories of at least one-third per customary serving or make a label claim of "cholesterol-free". (In this regard, see, for example, the list of standard serving sizes for various foods published in Food Labelling; Serving Sizes, 55 Fed. Reg. 29517 (1990) (to be codified at 21 C.F.R. 101.12), the disclosure of which is incorporated herein by reference, and the restrictions on labelling "cholesterol-free" at Food Labelling; Definitions of the Terms Cholesterol Free, Low Cholesterol and Reduced Cholesterol, 55 Fed. Reg. 29456 (1990)). It should also be noted that the fat removed from a particular formulation may be replaced with an equal amount by weight of an aqueous dispersion of starch precipitate, but that such equality may not be necessary or desirable in all instances. Further, it may be desirable to remove fat and add another ingredient (e.g. a gum, polydextrose, a protein, etc.) along with the aqueous dispersion of starch precipitate.

While this invention is generally directed to the replacement of fat and/or oil in a food formulation, it is of course within the contemplation of this invention that a debranched amylopectin starch will be used in an entirely new formulation to which it contributes fat-like organoleptic qualities but is not, in the strictest sense, replacing a pre-existing fat or oil ingredient. Moreover, it is contemplated that the debranched amylopectin starch will have utility as a thickener, bodying agent, or the like in foods that normally do not have a significant fat or oil component.

In general, the debranched amylopectin starch is incorporated into the food as an aqueous dispersion, typically comprised of a major amount (i.e. greater than 50% by weight) of water or other liquid medium and a minor amount (i.e. less than 50% by weight, typically 10% to 40%) of debranched starch solids. Alternatively, the isolated debranched amylopectin starch can be mixed with the food along with water and then subjected to dissolution and particle formation in those instances when the other ingredients of the food are capable of withstanding the conditions of dissolution, e.g. a salad dressing or imitation sour cream.

The debranched amylopectin starch can generally be heated while in a food system to dissolve a substantial portion of the particles. It appears that upon cooling, the debranched starch will reform into particles and the resulting food product displays acceptable organoleptic properties. In some food systems, however, reformation of the particles may be inhibited or modified by the presence of an ingredient that can interact with the solubilized debranched starch. If undesirable results will occur, a foodstuff containing the debranched amylopectin starch particles should not be subjected to conditions (e.g. elevated temperature) which will cause the particles (i.e. a majority by weight thereof) to dissolve. Accordingly, if the food formulation is to be cooked or otherwise heated to temperatures sufficient to dissolve the particles, such heating should be completed prior to the addition of the debranched amylopectin starch to the food. It should be noted, however, that in many foods that are cooked, e.g. cheesecake, the internal temperature and/or moisture availability may be insufficient to dissolve the starch particles.

As noted above, the terms "food" and "foodstuffs" are intended broadly, as relating to both nutritional and/or functional food ingredients. It is contemplated that one or more food ingredients may be mixed with the aqueous dispersion of debranched amylopectin starch precipitate, or even dry mixed with the debranched amylopectin starch prior to particle gel formation.

Among the food ingredients in the food formulations of this invention include flavors, thickeners (e.g. starches and hydrophilic colloids), nutrients (e.g. carbohydrates, proteins, lipids, etc. ), antioxidants, antimicrobial agents, non-fat milk solids, egg solids, acidulants, and so on.

Hydrophilic colloids can include natural gum material such as xanthan gum, gum tragacanth, locust bean gum, guar gum, algin, alginates, gelatin, Irish moss, pectin, gum arabic, gum ghatti, gum karaya and plant hemicelluloses, e.g. corn hull gum. Synthetic gums such as water-soluble salts of carboxymethyl cellulose can also be used. Starches can also be added to the food. Examples of suitable starches include corn, waxy maize, wheat, rice, potato, and tapioca starches.

Non-fat milk solids which can be used in the compositions of this invention are the solids of skim milk and include proteins, mineral matter and milk sugar. Other proteins such as casein, sodium caseinate, calcium caseinate, modified casein, sweet dairy whey, modified whey, and whey protein concentrate can also be used herein.

For many foods, it is accepted practice for the user to add the required amount of eggs in the course of preparation and this practice may be followed just as well herein. If desired, however, the inclusion of egg solids, in particular, egg albumen and dried yolk, in the food are allowable alternatives. Soy isolates may also be used herein in place of the egg albumen.

Dry or liquid flavoring agents may be added to the formulation. These include cocoa, vanilla, chocolate, coconut, peppermint, pineapple, cherry, nuts, spices, salts, flavor enhancers, among others.

Acidulants commonly added to foods include lactic acid, citric acid, tartaric acid, malic acid, acetic acid, phosphoric acid, and hydrochloric acid.

Generally, the other components of the various types of food formulations will be conventional, although precise amounts of individual components and the presence of some of the conventional components may well be unconventional in a given formulation. For example, the conventional other components for foods such as frozen desserts and dressings, are described in European Patent Publication No. 0 340 035, published November 2, 1989 (the pertinent disclosure of which is incorporated herein by reference), and the components and processing of table spreads is disclosed in U.S. Patent No. 4,869,919 (Lowery), the disclosure of which is incorporated by reference.

A particularly advantageous use of the debranched starch precipitates described herein may be the use thereof to replace a portion of the shortening used in a layered pastry article. In layered pastry articles (Danish, croissants, etc.), layers of a bread dough are assembled with a "roll-in" placed between the layers. The roll-in commonly contains a "shortening" (i.e. a fat and/or oil component) from an animal (e.g. butter) or vegetable (e.g. partially hydrogenated soybean oil) source. The assembled article, optionally containing a filling or topping, is then baked to form a finished pastry. At least a portion of the shortening of an otherwise conventional roll-in can be replaced with an aqueous dispersion of debranched amylopectin starch precipitate, preferably in admixture with an emulsifier (e.g. mono- and/or di-glycerides), and used to make a layered pastry.

The following examples will illustrate the invention and variations thereof within the scope and spirit of the invention will be apparent therefrom. All parts, percentages, ratios and the like are by weight throughout this specification and the appended claims, unless otherwise noted in context.

## EXAMPLES

## EXAMPLE 1

A totally debranched, waxy maize starch precipitate was prepared and fragmented as follows. Into a 3-liter stainless steel beaker was placed 2000 grams of aqueous slurry containing 5% dry solids waxy corn starch. The pH was adjusted to 4.5 using 0.5 N HCl and the beaker was placed in a 95°C water bath. The slurry was stirred and allowed to gelatinize and heat at 93-95°C for 20-30 minutes. The major portion of the resulting waxy starch paste (1150 grams paste) was placed into a pressure reactor and heated to 160°C with stirring. After stirring at 160°C for 30 minutes the waxy starch solution was cooled to 45°C and transferred to a 2-liter 3-neck round bottom flask equipped with stirrer, thermometer and a temperature controlled water bath. To the flask at 45°C and pH 4.5 was added 50 units isoamylase enzyme (Sigma Chemical Co., St. Louis, Missouri) per gram dry basis of starch. The enzyme reaction was allowed to proceed with stirring at 45°C for 48 hours. At the end of this period, the solution was heated to boiling (approximately 100°C) to inactivate enzyme then cooled and evaporated to 20% solids using a rotary evaporator.

The resulting solution was allowed to set in a refrigerator to precipitate/crystallize. The resulting slurry was centrifuged at about 10,000 g-force RCF in a Sorvall Centrifuge (GSA rotor) for 20 minutes. The supernatant was decanted. The sediment was resuspended in water to the original 20% solids concentration volume, heated to boiling then cooled and again allowed to precipitate/crystallize on standing in a refrigerator. The resulting slurry was centrifuged as before and the sediment dried at 50°C on a stainless tray in a forced air oven. The yield of product was calculated to be 81.3% on a dry basis.

Three additional samples were prepared in a similar manner with minor variations in treatment. Portions of all four samples were combined and heated to boiling to solubilize almost all material present in the 20% solids preparation. The hot solution was filtered through Whatman No. 1 filter paper on a Buchner funnel and the clear filtrate was placed in a refrigerator overnight to precipitate/crystallize. The resulting mass was filtered using Whatman No. 1 filter paper on a Buchner funnel and the precipitated mass was washed with additional water. The resulting wet cake was dried on a stainless steel tray overnight in a forced air oven at 50°C. The dried product was ground to pass through a US #60 mesh sieve and bottled.

Into a 250 ml 3-neck round bottom flask was placed 65.0 grams of the dried product above (57.5 grams dry basis) and 106.7 grams of acidic aqueous solution containing 3.87 grams of 100% HCl (approximately 1 N HCl solution). The mixture was heated to 60°C in a water bath and stirred at 60°C for 24 hours. The mixture was adjusted to pH 4.5 with 4% NaOH and then centrifuged. The supernatant was discarded and the sediment resuspended in the same volume of water and centrifuged again. The wet cake was dried in a forced air oven.

A 20.0% solids creme of the above product was prepared by simply blending at full speed in a Waring blender at 60°C for approximately 8 minutes with the temperature controlled.

## EXAMPLE 2

Into a 3-liter, 3-neck round bottom flask equipped with stirrer, thermometer and temperature regulated water bath was placed 2200 grams of 5% solids gelatinized waxy maize starch paste, previously heated to 96°C for 30 minutes, heated to 160°C for 30 minutes, then cooled to room temperature. To the waxy maize paste at pH 6.6 was added 11,000 units (100 units/gram dry starch) of Novo Nortek Promozyme Pullulanase Enzyme. The mixture was stirred and maintained at 58-60°C for 24 hours. Samples were taken at 6 hour, 18 hour, and 24 hour reaction periods, heated to about 90°C for about 10 minutes to inactivate enzyme, then freeze dried and analyzed by gel permeation chromatography (GPC) molecular weight.

After 24 hours, the reaction mixture was heated to about 90°C to inactivate enzyme and the hot solution filtered through Whatman #2 filter paper in a Buchner funnel. The slurry was concentrated via rotary evaporator to 20% solids, then placed into a refrigerator at 4°C overnight.

The resulting precipitated mixture was centrifuged and the supernatant discarded. The sediment was resuspended in an equal volume of water, heated to about 90°C and allowed to precipitate in a refrigerator at 4°C overnight. The precipitated mixture was centrifuged and the supernatant was discarded. The sediment was dried on stainless steel trays at 35°C overnight. The dried product was ground to pass through a US #60 mesh sieve. Gel permeation chromatography (GPC) analytical results are reported below.

| Reaction Time (hr) | $M_w$ | $M_n$ | $M_w/M_n$ | Peak Molecular Weights | Per Cent Less Than 20,000 Molecular Weight |
|---|---|---|---|---|---|
| 6 | 15,400 | 1,186 | 12.9 | 75,224;2556;335 | 87.5 |
| 18 | 7,900 | 1,074 | 7.3 | 64,412;2637;335 | 94.3 |
| 24 | 6,747 | 1,014 | 6.7 | 62,000;2772;345 | 95.2 |

If a finding of >80% saccharides having less than 20,000 molecular weight is considered as substantially completely debranched, then it can be seen that complete debranching was achieved using pullulanase enzyme.

## EXAMPLES 3-7

A series of debranched starch products were prepared from waxy maize starch under conditions similar to those employed in Examples 1 or 2, with isoamylase or pullulanase, respectively. Variations in conditions employed and results are shown in Table 1.

## TABLE 1

**EXAMPLE**

| | 3 | 4 | 5 | 6 | 7 |
|---|---|---|---|---|---|
| % Conversion | 57 | 69 | 85 | 96 | 100 |
| Debranching enzyme (P - pullulanase) (I - isoamylase) | P | P | I | P | I |
| % Cold water solubles, powder | 99.2 | 29.6 | 30.6 | 37.7 | 24.7 |
| Yield stress, pascals | Too gelled | Too gelled | 1110 | 17 | 428 |
| % Cold water solubles, creme (gel) | 13.8 | 11.5 | - | - | - |
| DSC: Onset °C | 43 | 47 | - | 47 | 57 |
| enthalpy | 9.5 | 29.3 | - | 21.3 | 20.6 |
| Water immobilization ($sec^{-1}$ by $^{17}O$ NMR) | 209 | 353 | - | 115 | - |
| Ratio of 1,4:1,6 linkages (by NMR) | 31/1 | 120/1 | - | * | - |
| Concentration of starch (during hydrolysis) | 20 | 20 | 20 | 5 | 5 |
| Units of enzyme (per g starch) | 3.79 | 7.5 | >200 | 100 | 100 |
| Number of precipitations | 0 | 2 | 2 | 2 | 2 |
| Centrifugation | No | No | No | Yes | No |

\* No α-1,6 linkages detected

### EXAMPLES 8-12

The gel samples were prepared using the debranched starches of Examples 3-7 by one of two procedures. Examples 8 and 9 started with the dry powders from Examples 3 and 4, respectively. To prepare a creme, 15% solids suspensions were prepared then heated to about 90°C and held at 90°C with stirring for one minute wherein almost all the solid material appears to dissolve. In Examples 10, 11, and 12, where limited sample was available, 25% creme samples were prepared as in Example 1 from the dry powder of Examples 5, 6, and 7, respectively. The cremes were diluted to 15% solids by adding deionized water, then heated with stirring to 90°C and held at 90°C for one minute. As with the powdered starting materials of Examples 8 and 9, most of the solids in the samples appeared to dissolve on this treatment. All of the hot 15% solids solutions were allowed to cool to room temperature then stored in a refrigerator prior to being analyzed with a Bohlin Rheometer.

Dynamic strain sweep evaluations were performed using a model VOR Bohlin Rheometer, from Bohlin Rheologi, Inc., East Brunswick, New Jersey, on the gels prepared above. The strain sweep experiments were conducted with the creme products at 1 Hz frequency using a concentric cylinder geometry. During the test, strain was increased by changing oscillation amplitude and the dynamic elastic modulus (G′) was measured as a function of strain.

The G′ values correspond to the strength of the network structure in the creme. The creme displays linear viscoelasticity at very low strains., i.e. G′ is independent of strain. Its behavior becomes non-linear (G′ decreases as strain increases) at a certain critical strain where the material structure becomes more "deformable". A short or brittle material will display a transition from linear to non-linear viscoelasticity at a lower strain. If the transition occurs at a higher strain, this indicates a long and cohesive texture.

In Figure 1, it can be seen that the 57% debranched gel showed only a slight break in its network structure at somewhat less than 100 millistrain. This sample appeared to behave like a polymer gel where we would expect a long and cohesive texture. By contrast, however, the 15% solids gels from debranched waxy maize starch with 69% debranching or greater displayed structural breakdown at less than about 50 millistrain. The products containing 85% and 100% debranched material exhibited a transition at less than about 10 millistrain. These products thus showed particle gel rheology much like that of CRISCO™ hydrogenated vegetable shortening (Procter & Gamble Co., Cincinnati, Ohio). The results of Example 11 (prepared from the powder of Example 6) appeared to be anomalous, and thus are not included here.

In general, particle gel compositions deform at low strain values and usually do so with large changes in G′ values. By contrast, polymer network gels usually deform at high strain values and typically display relatively low changes in G′ values.

## EXAMPLE 13

A particle gel of debranched amylopectin starch can be prepared from the debranched starch of Example 7 as follows. A sample of 25 parts by weight of the dry debranched starch of Example 7 is mixed with 75 parts by weight of tap water and heated with stirring to 90°C and held at 90°C for several minutes. The hot solution is then allowed to cool to room temperature and then stored at about 5°C overnight.

## EXAMPLE 14

## SPOONABLE SALAD DRESSING

A spoonable dressing can be prepared from the creme of Example 13 as follows.

| Ingredients | %, wt. |
|---|---|
| **Part A** | |
| Water | 22.00 |
| ISOSWEET ® 100 high fructose corn syrup (Staley) | 17.00 |
| Vinegar, white, 100 grain | 10.00 |
| SWEETOSE ® 4300 corn syrup (Staley) | 5.00 |
| STAR-DRI ® 35R corn syrup solids (Staley) | 3.20 |
| DELTA 7393 starch (Staley) | 2.85 |
| Salt | 1.80 |
| Carboxymethyl cellulose 7MF (Aqualon) | 0.20 |
| Mustard powder (McCormick) | 0.10 |
| Titanium dioxide (Warner-Jenkinson) | 0.08 |
| Garlic powder | 0.05 |
| Onion powder | 0.05 |
| Paprika | 0.0125 |
| Calcium disodium EDTA | 0.0075 |
| **Part B** | |
| Creme of Example 13 (10% dry solids) | 27.40 |
| Soybean oil | 8.00 |
| Egg yolk, fresh | 2.00 |
| Lemon juice, single strength | 0.25 |
| Total | 100.00 |

Procedure

1. Place water, vinegar, ISOSWEET, and SWEETOSE into a steam jacketed, swept surface cooker.
2. Thoroughly blend the dry ingredients of Part A, then disperse them into the water/vinegar/syrup mixture with agitation. Continue mixing while heating with steam to 190°F. Maintain this temperature for 5 minutes (with mixing); then immediately cool to 90° F.
3. Transfer Part A to a Hobart bowl; add egg yolk, creme, and lemon juice. Mix at low speed for 5 minutes with paddle.
4. Slowly add soybean oil and mix for another 5 minutes.
5. Process through a colloid mill at a 0.026" setting. Pack off finished product.

**EXAMPLE 15**

**BUTTERMILK DRESSING**

A buttermilk dressing can be prepared from the creme of Example 13 as follows.

| Ingredients | %, wt. |
|---|---|
| Buttermilk, liquid, 1% fat | 30.00 |
| Water | 23.94 |
| Creme of Example 13 | 23.50 |
| STAR-DRI ® 10 maltodextrin (Staley) | 8.00 |
| Vinegar, white, 100 grain | 5.40 |
| Seasoning mix #962-2489 (Griffith Labs) | 5.00 |
| Buttermilk solids, Beatreme #983 (Beatrice) | 1.00 |
| Instant TENDER-JEL ® C starch (Staley) | 1.00 |
| Salt | 0.75 |
| Sugar | 0.65 |
| Carboxymethyl cellulose, 7 MF (Aqualon) | 0.40 |
| Titanium dioxide (Warner-Jenkinson) | 0.10 |
| Potassium sorbate | 0.08 |
| Sodium benzoate | 0.0725 |
| Garlic powder | 0.05 |
| Onion powder | 0.05 |
| Calcium disodium EDTA | 0.0075 |
| Total | 100.00 |

Procedure

1. Blend all dry ingredients together.
2. Place water and buttermilk into a Hobart mixing bowl. Disperse the dry ingredient blend into this water/buttermilk mixture; mix with a Hobart paddle for 10 minutes at low speed.
3. Add the creme and mix for 10 minutes at medium speed.
4. Add vinegar and mix for 1 minute.
5. Process through a colloid mill at a 0.02" setting. Pack off finished product.

## EXAMPLE 16

## FRENCH DRESSING

A French dressing can be prepared from the creme of Example 13 as follows.

12

| Ingredients | %, wt. |
|---|---|
| Creme of Example 13 | 35.00 |
| ISOSWEET® 100 high fructose corn syrup (Staley) | 25.00 |
| Water | 23.98 |
| Vinegar, white, 100 grain | 10.00 |
| Tomato paste, 24-26% solids | 3.50 |
| Salt | 1.50 |
| Seasoning mix #912-0135 (Griffith Labs) | 0.30 |
| MIRA-THIK® 468 starch (Staley) | 0.30 |
| Seasoning mix #F34037 (McCormick) | 0.10 |
| Xanthan gum, Keltrol TF (Kelco) | 0.10 |
| Guar gum #8/22 (TIC Gums) | 0.05 |
| Mustard powder | 0.05 |
| Potassium sorbate | 0.05 |
| Titanium dioxide (Warner-Jenkinson) | 0.04 |
| Paprika | 0.0225 |
| Calcium disodium EDTA | 0.0075 |
| Color, yellow FD&C #5/#6 | to suit |
| Total | 100.00 |

Procedure

1. Place water and ISOSWEET into a Hobart bowl. Blend all dry ingredients together and disperse into the water and ISOSWEET mixture. Mix at low speed and allow to hydrate for 5 minutes.
2. Add tomato paste, creme and vinegar. Mix for 5 minutes.
3. Process through a colloid mill at a 0.02" setting. Pack off finished product.

**EXAMPLE 17**

**DIJON DRESSING**

A Dijon vinaigrette dressing can be prepared from the creme of Example 13 as follows.

| Ingredients | %, wt. |
|---|---|
| Water | 38.90 |
| Creme of Example 13 | 25.00 |
| Vinegar, white, 100 grain | 14.00 |
| Sugar | 6.50 |
| Dijon mustard (McCormick) | 6.00 |
| Lemon juice, single strength | 6.00 |
| Salt | 2.35 |
| Spice blend #F30378 (McCormick) | 1.00 |
| Xanthan gum, Keltrol TF (Kelco) | 0.14 |
| Red bell pepper, dried (McCormick) | 0.05 |
| Potassium sorbate | 0.04 |
| Annatto extract (Warner-Jenkinson) | 0.0125 |
| Calcium disodium EDTA | 0.0075 |
| Total | 100.00 |

Procedure

1. Place water in Hobart bowl.
2. Blend together all dry ingredients except the spice blend and red pepper. Disperse the dry ingredient blend into the water and mix with a paddle at low speed for 5 minutes.
3. Add the creme, mustard, vinegar and lemon juice. Mix for 5 minutes at low speed.
4. Process through a colloid mill at a 0.02" setting.
5. Blend in spice blend and red pepper. Then pack off product.

**EXAMPLE 18**

**SOUR CREAM**

An imitation sour cream can be prepared from the creme of Example 13 as follows.

| Ingredients | %, wt. |
|---|---|
| Creme of Example 13 | 39.79 |
| Sour cream, 18-20% fat | 29.83 |
| Water | 23.41 |
| Non-fat dry milk, low heat (Land O'Lakes) | 5.97 |
| Lactic acid, 88% | 0.40 |
| Xanthan gum, Rhodigel (Rhone-Poulenc) | 0.20 |
| Salt | 0.20 |
| Sodium citrate, hydrous, fine granular (Pfizer) | 0.20 |
| Total | 100.00 |

Procedure

1. Add lactic acid to water, thoroughly mix at Speed 1 with a Hobart mixer equipped with a wire whip.
2. Mix in sour cream and then all dry ingredients; mix until the mixture is uniform.
3. Blend in the creme to form a semi-homogeneous mixture.
4. Homogenize for smoothness.
5. Pack off and refrigerate until ready to use.

## EXAMPLE 19

## DEBRANCHED HIGH AMYLOSE STARCH

A 55% amylose corn starch (HI-SET C) is made up to 25% solids then jet cooked at 160°C with a retention time of 10 minutes at 160°C then cooled to ~100°C. The pH of the solution is adjusted to pH 6.0. Novo thermostable pullulanase enzyme as described in WO 92/02614 is added at 50 units per gram of starch and the reaction is allowed to proceed at 100°C for 24 hours, at which time GPC analysis will show that less than 10% of the remaining amylose and amylopectin molecules are above about 100,000 molecular weight.

The debranched solution is treated with 3% w/w (weight by weight basis) of decolorizing carbon (based on starch dry substance weight) at 90°C. The colorless carbon treated solution is cooled to 5°C for 16 hours to bring about crystallization. The crystallized mass is dried in a spray drier at 15% solids after dilution with water.

The spray dried material is made up to 20% solids and heated from 50°C to 100°C at 0.05°C/minute then cooled to 100°C at 0.05°C/minute. The heat treated material is treated at 20% solids at 80°C for 24 hours in 0.2 N HCl then cooled to room temperature. The acidic slurry is adjusted to pH 4.5 with 10% NaOH and microfiltered to reduce the soluble saccharide content to less than 10% (measured at room temperature). The retentate slurry is spray dried at about 15% solids to give a heat stable, starch based fat replacer.

A gel is prepared from the dried product above by slurrying the fat replacer powder at 15% solids then heating to 90°C and holding at 90°C with stirring one minute wherein almost all the material dissolves. The solution is then cooled to about 4°C then stored at 4°C for about 10 hours or longer during which time a gel is formed. The gel may then be utilized in various food formulations as a fat replacing composition.

## Claims

1. A foodstuff having a reduced level of fat and/or oil comprising a mixture of a foodstuff and a particle gel comprising a minor amount of debranched amylopectin starch particles dispersed within a major amount of an aqueous liquid as a replacement for at least a substantial portion of the fat and/or oil of said foodstuff.

2. A foodstuff of Claim 1 wherein said particle gel exhibits a transition in dynamic elastic modulus versus shear strain from substantially constant dynamic elastic modulus to decreasing dynamic elastic modulus, said transition being exhibited at a shear strain of less than about 50 millistrain.

3. A foodstuff of Claim 1 wherein said debranched amylopectin starch is derived from starch from a variety of Zea mays.

4. A foodstuff of Claim 1 wherein said debranched amylopectin starch is derived from a starch consisting essentially of amylopectin.

5. A foodstuff of Claim 1 wherein said debranched amylopectin starch is derived from waxy maize starch.

6. A foodstuff of Claim 1 wherein said debranched amylopectin starch is composed of more than about 80% by weight short chain amylose.

7. A foodstuff of Claim 1 wherein said debranched amylopectin starch is essentially free of amylopectin having a molecular weight in excess of 20,000 g/mol.

8. A method of formulating a foodstuff containing a fat and/or oil ingredient comprising replacing at least a substantial portion of said fat and/or oil ingredient with a particle gel comprising a minor amount of debranched amylopectin starch particles dispersed within a major amount of an aqueous liquid.

9. A method of Claim 8 wherein said particle gel exhibits a transition in dynamic elastic modulus versus shear strain from substantially constant dynamic elastic modulus to decreasing dynamic elastic modulus, said transition being exhibited at a shear strain of less than about 50 millistrain.

10. A method of Claim 8 wherein said debranched amylopectin starch is as defined in any one of claims 3 to 7.

11. A method of making a composition of matter useful in replacing fat and/or oil in a food formulation comprising dissolving a minor amount of debranched amylopectin starch in a major amount of an aqueous liquid and then forming particles of said debranched amylopectin starch, said dissolving and forming being effective to form a particle gel of said composition.

12. A method of Claim 11 wherein said particle gel exhibits a transition in dynamic elastic modulus versus shear strain from substantially constant dynamic elastic modulus to decreasing dynamic elastic modulus, said transition being exhibited at a shear strain of less than about 50 millistrain.

13. A method of Claim 11 wherein said debranched amylopectin starch is as defined in any one of claims 3 to 7.

14. An aqueous dispersion useful as a replacement for fats and/or oils comprising a particle gel comprised of a major amount by weight of an aqueous liquid and a minor amount by weight of debranched amylopectin starch particles dispersed therein.

15. An aqueous dispersion of Claim 14 wherein said particle gel exhibits a transition in dynamic elastic modulus versus shear strain from substantially constant dynamic elastic modulus to decreasing dynamic elastic modulus, said transition being exhibited at a shear strain of less than about 50 millistrain.

16. An aqueous dispersion of Claim 14 wherein said debranched amylopectin starch is as defined in any one of claims 3 to 7.

17. A method of making a composition of matter useful in replacing fat and/or oil in a food formulation comprising:
(a) gelatinizing a starch having an amylose content of less than about 40% by weight;
(b) debranching the amylopectin in said gelatinized starch in a debranched medium to convert more than about 80% by weight of the amylopectin to short chain amylose and form a debranched amylopectin starch in said medium;
(c) isolating said debranched amylopectin starch from said medium and drying said debranched amylopectin starch to a microbiologically stable moisture content;
(d) dissolving a minor amount of said dried debranched amylopectin starch in a major amount of an aqueous liquid and then forming particles of said debranched amylopectin starch, said dissolving and forming being effective to form a particle gel of said composition.

18. An edible composition comprising a mixture of a foodstuff or foodstuff ingredient and a particle gel comprising a minor amount of debranched amylopectin starch particles dispersed within a major amount of an aqueous liquid.

19. A composition as claimed in claim 18 wherein said particle gel exhibits a transition in dynamic elastic modulus versus shear strain from substantially constant dynamic elastic modulus to decreasing dynamic elastic modulus, said transition being exhibited at a shear strain of less than about 50 millistrain.

20. A composition as claimed in claim 18 wherein said debranched amylopectin starch is as defined in any one of claims 3 to 7.

21. A method of producing an edible composition as claimed in any one of claims 18 to 20 comprising admixing a foodstuff or foodstuff ingredient with a particle gel said particle gel comprising a minor amount of debranched amylopectin starch particles dispersed within a major amount of an aqueous liquid.

22. A method as claimed in claim 21 wherein said particle gel exhibits a transition in dynamic elastic modulus versus shear strain from substantially constant dynamic elastic modulus to decreasing dynamic elastic modulus, said transition being exhibited at a shear strain of less than about 50 millistrain.

23. A method as claimed in claim 21 wherein said debranched amylopectin starch is as defined in any one of claims 3 to 7.

24. A method of producing a fat and/or oil replacement foodstuff additive, said method comprising dissolving a minor amount of debranched amylopectin starch in a major amount of an aqueous liquid and then forming particles of said debranched amylopectin starch, said dissolving and forming being effective to form a particle gel.

25. A method as claimed in claim 24 wherein said particle gel exhibits a transition in dynamic elastic modulus versus shear strain from substantially constant dynamic elastic modulus to decreasing dynamic elastic modulus, said transition being exhibited at a shear strain of less than about 50 millistrain.

26. A method as claimed in claim 24 wherein said debranched amylopectin starch is as defined in any one of claims 3 to 7.

27. An edible fat and/or oil replacement composition comprising a particle gel comprised of a major amount by weight of an aqueous liquid and a minor amount by weight of debranched amylopectin starch particles dispersed therein.

28. A composition as claimed in claim 27 wherein said particle gel exhibits a transition in dynamic elastic modulus versus shear strain from substantially constant dynamic elastic modulus to decreasing dynamic elastic modulus, said transition being exhibited at a shear strain of less than about 50 millistrain.

29. A composition as claimed in claim 27 wherein said debranched amylopectin starch is as defined in any one of claims 3 to 7.

30. Use of a particle gel comprising a minor amount of debranched amylopectin starch particles dispersed within a major amount of an aqueous liquid in the manufacture of a foodstuff.

Figure 1

European Patent
Office

**EUROPEAN SEARCH REPORT**

Application Number

EP    92 30 7460

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| P,X | EP-A-0 486 936 (NATIONAL STARCH AND CHEMICAL INVESTMENT HOLDING CO.)<br>* claims 1-4,8-10 *<br>* page 3, line 30 - line 57 *<br>* page 7, line 54 - line 55 *<br>* example 4 *<br>--- | 1-30 | A23L1/09<br>C08B30/20 |
| A | WO-A-9 101 091 (ENZYTECH)<br>* page 3, line 7 - line 18 *<br>--- | 1-30 | |
| A | WO-A-9 107 106 (ENZYTECH)<br>* claims 1,2,7,14 *<br>* page 7, line 22 - line 31 *<br>* page 13, line 11 - page 14, line 8 *<br>* examples 10,11 *<br>--- | 1-30 | |
| D,A | EP-A-0 372 184 (NATIONAL STARCH AND CHEMICAL CO.)<br>* claims 1,3,6,8,12 *<br>* page 2, line 52 - page 3, line 44 *<br>& US-A-4 971 723<br>----- | 1-30 | **TECHNICAL FIELDS SEARCHED (Int. Cl.5 )**<br><br>A23L<br>C12P<br>C08B |

The present search report has been drawn up for all claims

| Place of search | Date of completion of the search | Examiner |
|---|---|---|
| THE HAGUE | 30 OCTOBER 1992 | VUILLAMY V.M.L. |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P0401)